# EUROPEAN PATENT APPLICATION

(11) **EP 4 728 976 A1**
(43) Date of publication of application: **22.04.2026**
(21) Application number: 24206576.1
(22) Date of filing: 15.10.2024
(51) Int. Cl.: A61B 5/02, A61B 5/021, A61B 5/026, A61B 5/0295, A61B 5/024

(54) **SYSTEM FOR DETERMINING PULSE TRANSIT TIME, PTT, OF A SUBJECT**

(71) Applicant: Stichting IMEC Nederland, 5656 AE Eindhoven (NL)
(72) Inventor: LORATO, Ilde Rosa, 5653 JG Eindhoven (NL); HERMELING, Evelien, 6027 NT Soerendonk (NL)
(74) Representative: AWA Sweden AB

(57) **Abstract**

There is provided a system for determining a pulse transit time, PTT, of a subject. The system comprises:
at least one light source to generate laser light illuminating at least one region of the subject, forming a speckle pattern on the region;
at least one imaging detector to acquire images of the speckle pattern; and
a processing unit to process the images.

The light source and the imaging detector generate images representing speckle vibrometry (SV) data and images representing speckle plethysmography (SPG) data. The processing unit extracts the SV data from the images and processes the SV data for determining a first time point representing a flow of blood entering an aorta. The processing unit extracts and processes the SPG data for determining a second time point representing a blood pulse arrival in a location of an artery system associated with the region. The processing unit determines the PTT based on the first and second time points.

## Description

### Technical field

The present disclosure relates to optical analysis for determining pulse transit time, PTT, and more specifically to system and a method for determining PTT of a subject based on speckle pattern analysis.

### Background

Optical measurement techniques have found widespread application within the medical field and may provide information on parameters such as heart rate (HR), heart rate variability (HRV), blood pressure, blood flow, blood volume, cardiac output, arterial elasticity, respiration rate, oxygen saturation, venous and arterial flow pressure motion.

Pulse Transit Time (PTT) and Pulse Wave Velocity (PWV) are important parameters to assess the cardiovascular health of subjects. These parameters may for example provide information about arterial stiffness. PTT in particular may also be used as an indicator for continuous blood pressure. PTT may be measured by analyzing the time delay of a pressure wave in two different locations, typically a proximal and a distal location, of the subject. Photoplethysmography (PPG) is an optical measurement technique with which measurements of volume change may be made, as the blood flows through the blood vessels. PPG is known to be used to measure the occurrence of the pressure wave of the subject, for example in combination with an electrocardiogram (ECG), in order to estimate PTT.

However, PPG suffers from drawbacks having a negative influence on the accuracy of the measurements of the times at which the pressure wave arrives.

Further, PPG may perform poorly in low perfusion conditions and may be challenging to use with darker skin tones.

Hence, there is a need in the art for further improvements related to optical analysis for determining PTT.

### Summary

An objective of the present disclosure is to mitigate, alleviate or eliminate one or more of the above-identified deficiencies in the art and disadvantages singly or in any combination. These and other objectives are at least partly met by the invention as defined in the independent claims. Preferred embodiments are set out in the dependent claims.

According to a first aspect there is provided a system for determining a pulse transit time (PTT) of a subject, the system comprising:
at least one light source configured to generate laser light for illuminating at least one region of the subject, wherein the laser light is configured to form a speckle pattern on the at least one region;
at least one imaging detector configured to acquire images of the speckle pattern; and
a processing unit configured to receive and process the images of the speckle pattern from the at least one imaging detector;
wherein the at least one light source and the at least one imaging detector are configured to generate images representing speckle vibrometry (SV) data, and wherein the processing unit is configured to extract the SV data from the images and process the SV data for determining a first time point representing a flow of blood entering an aorta;
wherein the at least one light source and the at least one imaging detector are configured to generate images representing speckle plethysmography (SPG) data, and wherein the processing unit is configured to extract the SPG data from the images and process the SPG data for determining a second time point representing a blood pulse arrival in a location of an artery system associated with the at least one region; and
wherein the processing unit is further configured to determine the PTT based on the first time point and the second time point.

By the term "speckle pattern" is here meant an interference pattern formed when coherent light, such as laser light, illuminates a surface. On a static surface, the speckle pattern will be static and form bright and dark dots due to constructive and destructive interference of light. Speckle patterns may be formed when light from a light source is reflected and/or scattered by the surface. In the present context, the laser light illuminates at least one region of the subject, to form a speckle pattern thereon. The at least one region is typically a surface of living biological tissue of the subject, such as the skin of the subject or facial hair of the subject. However, it should be realized that the at least one region may alternatively be a piece of clothing worn by the subject.

By the term "light source" is here meant any unit, device and/or element at which light is generated. By way of example, the light source may be, but is not limited to a laser or a laser diode. In this context the term "light" should be allowed a broad interpretation, not limited to visible electromagnetic radiation. Rather, the term "light" may also include for example ultra-violet light and infra-red light.

Laser light is typically at least partially coherent light. Coherent light may be advantageous as it improves the interference visibility of the speckle pattern.

The at least one light source may be one light source, two light sources or more light sources. By way of example, the at least one light source may be a single light source configured to form the speckle pattern for the speckle vibrometry (SV) data and to form the speckle pattern for the speckle plethysmography (SPG) data. By way of further example, the at least one light source may alternatively comprise a first light source configured to form the speckle pattern for the speckle vibrometry (SV) data, and a second light source configured to form the speckle pattern for the speckle plethysmography (SPG) data.

By way of example, the at least one light source may be configured to form these speckle patterns simultaneously or alternatingly.

By the term "imaging detector" is here meant any unit or device onto which a plurality of separate light sensitive elements are arranged such that they may individually detect the light intensity impinging onto the respective light sensitive elements, and in response thereof produce an electrical signal. The light sensitive elements on the imaging detector may be arranged along one or more rectilinear lines. By way of example, an imaging detector may be, but is not limited to, a charge-coupled device (CCD) and complementary metal oxide semiconductor (CMOS).

The at least one imaging detector may be one imaging detector, two imaging detectors or more imaging detectors. By way of example, the at least one imaging detector may be a single imaging detector configured to acquire images of the speckle pattern for the speckle vibrometry (SV) data and to acquire images of the speckle pattern for the speckle plethysmography (SPG) data. By way of further example, the at least one imaging detector may alternatively comprise a first imaging detector configured to acquire images of the speckle pattern for the speckle vibrometry (SV) data, and a second imaging detector configured to acquire images of the speckle pattern for the speckle plethysmography (SPG) data.

By way of example, the at least one imaging detector may be configured to acquire images of these speckle patterns simultaneously or alternatingly.

The processing unit may be implemented as a general-purpose processing unit, such as a central processing unit (CPU), which may execute the instructions of one or more computer programs in order to implement the processing of the images. The processing unit may alternatively be implemented as firmware arranged in an embedded system, or as a specifically designed processing unit, such as an Application-Specific Integrated Circuit (ASIC) or a Field-Programmable Gate Array (FPGA), which may be configured to implement the processing of the images. The processing unit may optionally comprise a hardware accelerator.

The processing unit may be arranged internally in the system. Alternatively, the processing unit may be arranged in an external unit, or it may be a processing unit of an external computer not being part of a common physical housing holding the system. Data may be transferred to the processing unit, either by a wired connection or wirelessly. As yet another alternative, the processing unit may be distributed among physical units, such that parts of the processing may be performed in different physical units.

Speckle Vibrometry (SV), also alternatively referred to as Laser Speckle Vibrometry, may be used for monitoring sound and/or vibrations. SV is based on monitoring a defocused speckle pattern to analyze the speckles translational movement caused by vibrations, by illuminating a region with a coherent light source and acquiring images of the speckle pattern. By way of example, images of defocused speckles may be acquired by using an imaging lens being defocused with respect to the speckle pattern. Heart sounds of a living subject may cause subtle vibrations. Said vibration may be detectable at for example the chest region of the subject. These vibrations may be detectable with SV when monitoring the defocused speckles. From the acquired images, SV data may be extracted and analyzed by the processing unit. A time point at which a movement occurs may be the time point representing the flow of blood entering the aorta of the subject. This may thus represent the first time point.

By way of example, the at least one region of the subject being illuminated by the light source may be a region of the skin of the subject. However, it should be realized that the at least one region may alternatively be a piece of clothing worn by the subject, as for example a shirt. Put differently, vibration related to the heart may thus be detectable also in SV data acquired on clothing worn by the subject.

It serves to mention that a blood flow that enters the aorta may be represented in a number of different manners. By way of example, a blood flow entering the aorta may be represented by vibrations from the opening of the aortic valve, from contraction of the heart, and/ or from the expansion of the aortic root.

Speckle Plethysmography (SPG) is based on monitoring changes in the speckle contrast, by illuminating a region with a coherent light source and acquiring images of the speckle pattern. Living biological tissue such as a region of the skin of the subject presents movements of the tissue, caused by for example blood flow through the tissue and contraction and dilatation of blood vessels in the tissue in the region. When the region of skin of the subject is illuminated by coherent light, the speckle pattern may be temporarily perturbed by this movement, such that the speckle pattern is blurred, which may result in reduced speckle contrast of the speckle pattern. From the acquired images, SPG data may be extracted and analyzed by the processing unit. A time point at which a reduction in the speckle contrast occurs may be the time point representing the blood pulse arrival in the location of the artery system at the illuminated region. This may thus represent the second time point.

It should be realized that as alternatives to speckle contrast, speckle counting and/or averaging of a spatial Fourier transform of the data may be used as an indication of the blood pulse arrival.

Images representing SPG data may be acquired by the imaging detector by comprising also an imaging lens to be in focus with respect to the speckle pattern. However, it is conceivable that images of SPG data may alternatively be acquired by not using an imaging lens, as for example by lens-free imaging.

It serves to mention that the term "plethysmography" generally refers to measurements of changes in volume. By way of example, in conventional Photoplethysmography (PPG), not involving speckles, contraction and dilatation of blood vessels as the blood flows through the blood vessels is measured. PPG thus performs a measurement of volume change. However, in Speckle Plethysmography (SPG), wherein speckle contrast is measured, the measurement may be affected also by the movements caused by the blood flow. Therefore, although the term SPG implies measurements of volume change, it should be understood that an SPG signal does not necessarily result solely from volume change but may additionally or alternatively result from other changes of properties such as movements of the tissue and/or changes of scattering properties.

By the present arrangement, images representing SV data and images representing SPG data may be acquired. The SV data and the SPG data may be acquired by illuminating and monitoring at at least one region. Given as a non-limiting example, the SV data and the SPG data may be acquired at two different regions of the subject, i.e. a first region and a second region. The first region and the second region may be a proximal region and a distal region, respectively. Alternatively, SV data and the SPG data may be acquired at the same single region of the subject, typically being the proximal region. It serves to mention that the proximal region may typically be proximal with respect to the heart. By way of example, the proximal region may be located on the chest of the subject. In this manner, the first time point and the second time point may be determined, wherein the first time point represents the flow of blood entering the aorta of the subject, and a second time point represents the blood pulse arrival in the location of the artery system at the illuminated region. In other words, by using imaging of speckles, two different phenomena may be monitored. The first time point may be determined by detecting vibration, or sound, propagating from the heart to the illuminated region. The second time point may be determined by detection of the arrival of the blood flow in the illuminated region. Since the speed of sound propagating in the body of the subject is significantly faster than the speed at which the blood travels, the first and the second time points may be different, even if detection is made at the same single region. Based on the first and second time points, the pulse transit time (PTT) may be determined. By way of example, the PTT may be determined by determining a difference between the first and second time points.

An advantage is that by imaging speckle patterns, two different phenomena may be monitored, namely vibrations, or sound, from the heart, and the passage of blood in the region. By the present arrangement, the first time point may be related to the sound of the heart and the second time point may be related to the arrival of the blood flow at the region. In this manner, PTT may be reliably determined. Consequently, a system for determining PTT may be provided, allowing monitoring of a single region of the subject, without the requirement of monitoring at a proximal and at a distal region. It serves to mention that monitoring of a single region for determining PTT would not be possible in case passage of the blood were to be determined at two different regions. This provides flexibility to how the system is configured, allowing a number of different embodiments to be used.

By way of example, the system may be configured so that the parts for the SV and the SPG are separate, e.g. having separate light sources and/or separate imaging detectors. By the present arrangement, SV data and SPG data may be acquired in parallel. Such a system may further be configured to have different properties for SV and SPG, as for example using different light wavelengths. This, in turn, may allow for easier separation of the signals, e.g. by filtering, thereby reducing the risk of cross-talk between the SV and SPG data.

Alternatively, this allows for a more compact system to be provided, since illumination and imaging of only a single region needs to be covered, instead of illuminating and imaging two separate regions being spatially separated by a large distance. It further allows for all parts of the system to be arranged within a single small and compact housing. Requirements of two separate regions being spatially separated by a large distance is a known drawback in the prior art, for example in conventional PPG based determination of PTT. Further, the combination of SV data and SPG data for determining PTT may provide the advantage that the same light source and/or imaging detector may be used for both sets of data, providing an even more compact solution. In such a solution, images for the SV and SPG may be acquired alternatingly by for example switching between different configurations of the system.

Another advantage is that both SV data and SPG data is less sensitive to changes of ambient lighting, whereas conventional PPG is more sensitive and thus more affected by changes in ambient lighting. In that respect, a more robust system for determining PTT may be provided, that may be used at various location, without requirements to the ambient lighting.

Yet another advantage is that a system may be provided allowing a more precise determination of PTT to be made. SV data and SPG data acquired as described above may provide features with greater sharpness, as for example peaks representing the first and second time points, as compared to the more blunted morphology of conventional PPG data.

Yet another advantage is that a system for determining PTT may be provided, which is less affected by the level of skin melanin of the subject. For example, conventional PPG based determination of PTT is affected by the skin melanin, especially at high levels. Both the SV data and the SPG data show less sensitivity to skin melanin levels.

According to an embodiment, the at least one light source and the at least one imaging detector are further configured to generate images representing photoplethysmography (PPG) data, and wherein the processing unit is configured to extract the PPG data from the images and process the PPG data for determining a third time point. By way of example, the third time point may represent a blood pulse arrival in a location of an artery system associated with the at least one region. By way of further example, the third time point may represent a blood pulse arrival in a location of a capillary system associated with the at least one region. The processing unit may be further configured to determine a PTT based on at least two of the first time point, the second time point, or the third time point. Put differently, the system for determining a PTT of a subject may be configured to acquire SV data, SPG data, and PPG data.

According to an embodiment, the at least one light source and the at least one imaging detector are configured to generate the images representing the SV data, in a first configuration of the system, and
wherein the at least one light source and the at least one imaging detector are configured to generate the images representing the SPG data, in a second configuration of the system.

In the present manner, the first configuration of the system may be configured to provide different properties of the system than provided by the second configuration. The properties may thus be provided to be suitable for SV and SPG, respectively. By way of example, the first configuration may provide a defocused imaging lens for acquisition of the SV data, whereas the second configuration may provide an imaging lens being in focus with respect to the speckle pattern for acquisition of the SPG data. By way of further example, the first configuration may provide a collimated laser light illumination for SV, whereas the second configuration may provide a divergent laser light illumination for SPG.

The first configuration and the second configuration may be two separate sets of components. Alternatively, the first configuration and the second configuration may share at least some of the components, such as the at least one light source and/or the at least one imaging detector. The system may be configured to switch between different properties, as for example switching between collimated and divergent laser light for illumination, focused and defocused imaging.

Images representing the SV data and images representing the SPG data are thus separate images. Thus, two separate image sets may be acquired, one image set representing the SV data and another image set representing the SPG data.

An advantage of this embodiment is that, in case some of the components are shared, the number of components may be reduced, and the system may be both more compact and may be produced at a lower cost.

According to an embodiment, in the first configuration, the at least one light source is configured to generate collimated laser light for illuminating the at least one region of the subject to form the speckle pattern for the speckle vibrometry (SV) data.

Collimated laser light is well suited for forming the speckle pattern for the SV data.

An advantage with this embodiment is that collimated laser light may provide enhanced selectivity in the vibrations that are monitored.

However, it serves to mention that divergent laser light may alternatively be used for SV.

According to an embodiment, in the second configuration, the at least one light source is configured to generate divergent laser light for illuminating the at least one region of the subject to form the speckle pattern for the speckle plethysmography (SPG) data.

Divergent laser light is well suited for forming the speckle pattern for SPG data.

An advantage with this embodiment is that divergent laser light may provide illumination for a large region of the subject, which in turn may provide a speckle pattern imaged onto a large number of light sensitive elements. By the present arrangement, a system with improved signal to noise ratio may be provided.

However, collimated laser light may alternatively be used for SPG. By such an arrangement, illumination of a small region of the subject may be provided. In order to monitor the speckle pattern, the at least one imaging detector may be provided with magnification means.

According to an embodiment, in the first configuration, the at least one imaging detector comprises an imaging lens configured to be defocused with respect to the speckle pattern.

Using an imaging lens is an easy manner of providing defocused images of the speckle pattern. Defocused images of the speckle pattern is well suited for determining the speckles translational movement caused by vibrations, for SV. In case the speckle pattern is imaged in focus, the speckles may more randomly due to the vibrations. However, when using a defocused imaging lens the speckles may show a translational movement. Such translational movements may facilitate monitoring of the speckle movements.

According to an embodiment, in the second configuration, the at least one imaging detector comprises an imaging lens configured to be in focus with respect to the speckle pattern.

Imaging lenses are readily available in a large variety. Using an imaging lens is therefore an easy manner of providing in-focus images of the speckle pattern.

According to an embodiment, the at least one light source is a single light source,
wherein the single light source is configured to form the speckle pattern for the speckle vibrometry (SV) data, and
wherein the single light source is configured to form the speckle pattern for the speckle plethysmography (SPG) data.

In case a single light source is used for both SV and SPG, it is conceivable that the same laser wavelength may also be used for both SV and SPG. However, it is equally conceivable that different wavelengths may be used for SV and SPG. By way of example, the single light source may be a tunable light source, as for example a tunable laser. The tunable light source may be configured to dynamically change the wavelength of the output light. In this manner, the illumination for SV may be provided with a wavelength having lower penetration depth, and the illumination for SPG may be provided with a wavelength having higher penetration depth. By way of further example, the single light source may be a white light source, such as a white laser or a super luminescent diode. The output wavelength may be selected by for example, rotating filters or tunable metasurfaces, to provide different wavelengths for the SV and SPG illumination.

An advantage with using a single light source for illumination for both the SV data and the SPG data is that the size of the system may be reduced. Thus a compact system may be provided. Further, the cost of the system may be reduced, since light sources may be an expensive part of the system.

Another advantage with using a single light source for illumination for both the SV data and the SPG data is that the power consumption of the system may be reduced. A significant part of the power consumption of the system may be come from the light sources. Thus, by reducing the number of light sources to a single light source may have a significant impact on the power consumption.

According to an embodiment, the single light source is configured to switch between, in the first configuration, generating divergent laser light for illuminating the at least one region of the subject to form the speckle pattern for the speckle vibrometry (SV) data, and, in the second configuration, generating collimated laser light for illuminating the at least one region of the subject to form the speckle pattern for the speckle plethysmography (SPG) data.

Given as a non-limiting example, a dynamically adaptable lens may be arranged in the laser light path from the single light source, such that the light source may provide collimated laser light for SV illumination and divergent laser light for SPG illumination. Alternatively, a microlens array or tunable metasurfaces may be used for this purpose.

According to an embodiment, the at least one light source comprises a first light source and a second light source,
wherein the first light source is configured to form the speckle pattern for the speckle vibrometry (SV) data, and
wherein the second light source is configured to form the speckle pattern for the speckle plethysmography (SPG) data.

By the present arrangement, the first light source and the second light source may be provided with different properties suitable for the generation of the speckle pattern for SV data and for generating the speckle pattern for SPG data, respectively. By way of example, the first light source may provide a collimated laser light illumination for SV, whereas the second light source may provide a divergent laser light illumination for SPG.

The first light source and the second light source may be configured to generate laser light of different wavelengths, thereby allowing the use of two different wavelengths for the SV data and the SPG data. The present arrangement may reduce the risk of cross-talk between the SV images and the SPG images. Additionally, using two different wavelength allows for filtering of the respective signals to be made, in order to prevent light of unwanted wavelengths to reach the respective imaging detector, further reducing the risk of cross-talk.

It serves to mention that the first light source used for SV data may preferably have a wavelength with a low penetration depth into the subject. The second light source used for SPG data may preferably have a wavelength with a high penetration depth. The penetration depth may be related to the level of absorption by water of the wavelength in question.

An advantage with this embodiment is that cross-talk between the SV data and the SPG data may be reduced or even eliminated. Thus, in turn, may improve the quality of the images of the respective speckle patterns, and thus improved accuracy of the respective measurements.

Another advantage is that it may be easy to illuminate two regions of the subject being spatially separated. If two separate light sources are used, the first light source may easily illuminate the first region and the second light source may illuminate the second region, since the first and second light sources may be separate units, which may be moved independently with respect to one another.

According to an embodiment, the at least one imaging detector is a single imaging detector,
wherein the single imaging detector is configured to acquire images representing speckle vibrometry (SV) data, and
wherein the single imaging detector is configured to acquire images representing speckle plethysmography (SPG) data.

Given as a non-limiting example, a single imaging detector being shared between the SV and SPG image acquisition may be realized by providing microlens array onto the single imaging detector. The microlens array may defocus part of the single imaging detector for the SV data and may focus another part of the single imaging detector for the SPG data.

Given as another non-limiting example, the single imaging detector may comprise an imaging lens configured to switch between being defocused with respect to the speckle pattern, and being in focus with respect to the speckle pattern. Such a dynamically adaptable imaging lens may be used to acquire the SV data and the SPG data alternatingly by the single imaging detector. An adaptable imaging lens may be realized by tunable metasurfaces to modulate the focusing properties of the imaging lens in time, or by an optical phased array to dynamically change the focusing properties of the imaging lens.

An advantage with using a single imaging detector for acquiring images representing both the SV data and the SPG data is that the size of the system may be reduced. Thus, a compact system may be provided. Further, the cost of the system may be reduced, since imaging detector may be an expensive part of the system.

Another advantage with using a single imaging detector for acquiring images representing both the SV data and the SPG data is that the power consumption of the system may be reduced.

According to an embodiment, the at least one region comprises a first region and a second region, spatially separated from the first region, wherein, in the first configuration, the at least one light source and the at least one imaging detector are configured to generate images of the speckle pattern in the first region representing speckle vibrometry (SV) data, and wherein, in the second configuration, the at least one light source and the at least one imaging detector are configured to generate images of the speckle pattern in the second region representing speckle plethysmography (SPG) data.

The first region and the second region may be a proximal region and a distal region, respectively. Since the first region and the second region are two separate regions, the locations of the first region and the second region may be selected independently. The locations may be selected such that the first region is a good location for acquiring images for SV data, and such that the second region is a good location for acquiring images for SPG data.

Since the first region is spatially separated from the second region, the distance between the first region and the second region may be freely selected. By way of example, the distance between the first region and the second region may be made large. More specifically, the distance between the second region and the location of the aorta of the subject may be made large.

An advantage with this embodiment is that the accuracy of the determination of the first time point and the second time point may be improved, since the regions may be selected to be well suited for the respective measurements. This may improve the overall accuracy of the determination of the PPT.

Another advantage with this embodiment is that a large distance between the aorta and the second region may further improve the accuracy of the determined PTT. With a large distance between the aorta and the second region, the time difference between the first time point and the second time point may be large. In this manner, the uncertainty in the determination of the first and second time point, respectively, may be small in relation to the total time difference, and thus in relation to the PTT.

According to an embodiment, the at least one region is a single region, wherein the at least one light source and the at least one imaging detector are configured to generate images of the speckle pattern in the single region representing speckle vibrometry (SV) data, and wherein the at least one light source and the at least one imaging detector are configured to generate images of the speckle pattern in the single region representing speckle plethysmography (SPG) data.

Thus, the SV data and the SPG data may be acquired at the same single region of the subject. By way of example, said single region may typically be the proximal region. In this manner, the first time point and the second time point may be determined, wherein the first time point represents the flow of blood entering the aorta of the subject, and a second time point represents the blood pulse arrival in the location of the artery system at the illuminated single region.

An advantage with this embodiment is that a more compact system to be provided, since illumination and imaging of only a single region needs to be covered, instead of illuminating and imaging two separate regions being spatially separated by a large distance. It further allows for all parts of the system to be arranged within a single small and compact housing.

According to an embodiment, the processing unit is configured to determine the PTT by determining a difference between the first time point and the second time point.

An advantage with this embodiment is that an easy and computationally efficient manner of determining the PTT may be provided.

According to an embodiment, the processing unit is further configured to determine a pulse wave velocity (PWV) of the subject, based on the first time point and the second time point.

Pulse wave velocity (PWV) is another important parameter to assess the cardiovascular health of the subject, e.g. arterial stiffness.

According to an embodiment, the system is configured for sensing at a distance and is configured to illuminate the at least one region of the subject and to acquire images of the speckle pattern from a distance to the subject in the interval of 0.1 m to 4 m.

It should be realized that the system may be configured to illuminate the at least one region and to acquire images of the speckle pattern from a distance within the size of a typical room in a hospital.

The system may be used to acquire the SV data and the SPG data at a distance from the subject to monitor for example vital signs such as PTT. By the present arrangement, no physical contact between the system and the subject is required.

By way of example, the system for sensing at a distance may be a system wherein the at least one light source, the at least one imaging detector, and the processing unit are arranged in a common housing. One or more of said parts may be integrated to form a compact system for sensing at a distance.

By way of further example, the system for sensing at a distance may alternatively be a system wherein one or more of the at least one light source, the at least one imaging detector, and the processing unit are arranged external to the other parts.

An advantage with a system for sensing at a distance is that monitoring of PTT may be provided without the requirement of physical contact between the system and the subject. This in turn may allow for monitoring to be made for a long time without causing any discomfort to the subject.

According to an embodiment, the system further comprises a housing configured to be wearable on a body of the subject, and wherein the at least one light source and the at least one imaging detector are arranged in or on the housing.

By way of example, the housing may comprise a textile/garment configured to be worn on the body, or the housing may comprise a belt or strap, configured to be worn around a body part of the body. By way of further example, the housing may be implemented in the form of a clamp configured to be clamped around a body part of the body.

An advantage with this embodiment is that it may provide a convenient manner of wearing the system, such that the system may be worn for a long time and thus providing monitoring for a long time, while minimizing discomfort for the subject.

It serves to mention that a system alternatively comprising a combination of wearable parts being in physical contact with the subject, and parts at a distance or stationary parts may also be conceivable. Given as a non-limiting example, the system may comprise a wearable part comprising the at least one light source and the at least one imaging detector arranged in a common housing, and having a wired connection to the processing unit being arranged for example in a stationary computer. Moreover, the wearable device may also comprise cables for supplying electrical power to the at least one light source and/or the at least one imaging detector. By the present arrangement, parts of the system may be wearable, however the system may not be portable since the subject may be restricted to wearing the system for example inside a room at a hospital, but may not be able to leave the room due to cabling being connected to stationary parts in the room.

According to an embodiment, the at least one light source and the at least one imaging detector are configured to generate images representing both the SV data and the SPG data in the same image, and wherein the processing unit is configured to extract the SV data and the SPG data from the same image.

It is conceivable that each image of the acquired images comprise both SV data and SPG data. The processing unit may be further configured to extract the SV data and the SPG data, respectively, from the images. The processing unit may thus be configured to separate the SV data from the SPG data and vice versa. By way of example, said separation may be provided by filtering, such as low-pass, high-pass, and/or band-pass filtering. By way of further example, said separation may be provided by wavelet filtering and/or wavelet decomposition in order to highlight one of the two signals. Given as further non-limiting examples, said separation may be provided by Blind Source Separation methods, such as Independent Component Analysis, or a combination of peak detection methods using time and amplitude assumptions. It serves to mention that a system configured for lens-free imaging may facilitate in providing focused information for SPG from defocused images for SV.

It serves to mention that the above mentioned approaches of separating the SV data and SPG data may be equally applicable when separate images sets for SV and SPG are acquired. For example, even when two separate image sets are acquired, cross-talk may occur between the SV and SPG. Thus, to reduce or even eliminate the effect of the cross-talk, the above mentioned approaches may be applied to separate or enhance the two sets of data.

According to a second aspect there is provided a method for determining a pulse transit time of a subject, the method comprising:
illuminating at least one region of the subject, by laser light generated by at least one light source of a system, wherein the laser light is configured to form a speckle pattern on the at least one region;
generating, by the at least one light source and at least one imaging detector, images representing speckle vibrometry (SV) data;
generating, by the at least one light source and the at least one imaging detector, images representing speckle plethysmography (SPG) data;
receiving, by a processing unit of the system, the images of the speckle pattern from the at least one imaging detector;
extracting, by the processing unit, the SV data from the images;
processing, by the processing unit, the SV data for determining a first time point representing a flow of blood entering an aorta;
extracting, by the processing unit, the SPG data from the images;
processing, by the processing unit, the SPG data for determining a second time point representing a blood pulse arrival in a location of an artery system associated with the at least one region; and
determining, by the processing unit, the PTT based on the first time point and the second time point.

Effects and features of the second aspect are largely analogous to those described above in connection with the first aspect. Embodiments mentioned in relation to the first aspect are largely compatible with the second aspect. It is further noted that the disclosure relates to all possible combinations of features unless explicitly stated otherwise.

Other objectives, features and advantages of the present disclosure will appear from the following detailed description, from the attached claims as well as from the drawings.

### Brief descriptions of the drawings

The above, as well as additional objects, features and advantages of the present disclosure, will be better understood through the following illustrative and non-limiting detailed description, with reference to the appended drawings. In the drawings like reference numerals will be used for like elements unless stated otherwise.
Fig. 1A schematically illustrates a system for determining a pulse transit time, PTT, at a distance of a subject.
Fig. 1B schematically illustrates the data processing performed by a processing unit of the system, by illustrating the SV data on the top row and the SPG data on the bottom row.
Fig. 2 schematically illustrates an alternative system for determining a pulse transit time, PTT, of a subject 10, which system comprises a first subsystem and a second subsystem.
Fig. 3A schematically illustrates a system for determining a pulse transit time, PTT, of a subject, which system is configured to acquire images of both the SV data and the SPG data in the same image.
Fig. 3B schematically illustrates data extracted from images comprising both the SV data and the SPG data in the same image.
Fig. 4 schematically illustrates a wearable system for determining a pulse transit time, PTT, of a subject.
Fig. 5 illustrates a schematic block diagram shortly summarizing the method for determining a pulse transit time, PTT, of a subject.

### Detailed description

In cooperation with attached drawings, the technical contents and detailed description of the present inventive concept are described thereinafter according to a preferable embodiment, being not used to limit the claimed scope. This inventive concept may be embodied in many different forms and should not be construed as limited to the embodiments set forth herein; rather, these embodiments are provided for thoroughness and completeness, and fully convey the scope of the inventive concept to the skilled person.

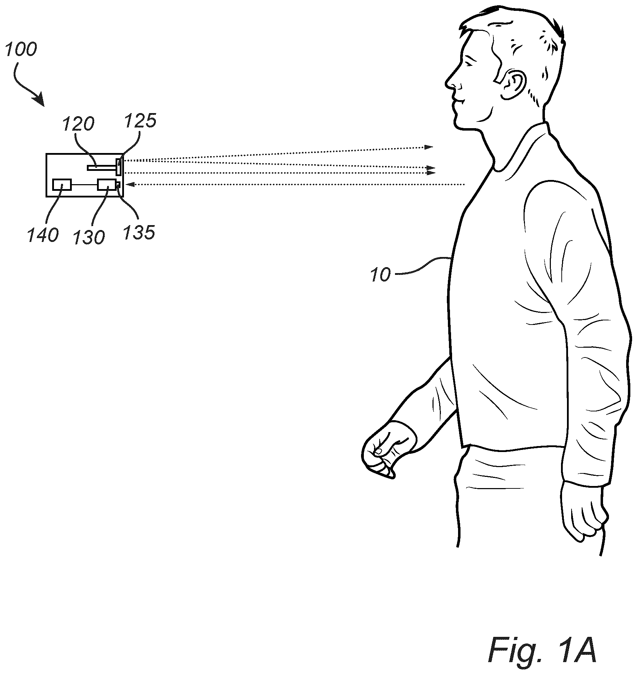

Fig. 1A schematically illustrates a system 100 for determining a pulse transit time, PTT, of a subject 10. The system 100 is here illustrated as a system configured for sensing at a distance to the subject 10, but it should be realized that system 100 may alternatively be configured to be wearable on the body of the subject 10, and thus to be in physical contact with the subject 10.

The system 100 is configured to use speckle vibrometry, SV, to determine when the flow of blood enters the aorta of the subject 10, and to use speckle plethysmography, SPG, to determine when a blood pulse arrives in a location of the artery system associated with a predetermined region of the subject 10.

The system 100 comprises a light source 120. The light source is configured to generate laser light for illuminating the region of the subject 10 from a distance. Typically, the distance between the system 100 and the subject 10 is in the interval of 0.1 m to 4 m, which corresponds to a typical distance range available in a room in a hospital. Upon illumination of the subject 10, the laser light forms a speckle pattern on the illuminated region.

In the present example, the light source 120 is configured to illuminate a single region of the subject 10, so as to form the speckle pattern for the SV data, and the speckle pattern for the speckle plethysmography SPG data in the same single region. Typically, this single region is a proximal region such as the chest region of the subject 10.

The system 100 has a first configuration with which the SV data is acquired, and a second configuration with which the SPG data is acquired.

The light source 120 may comprise an adaptable lens 125 arranged in the beam path of the laser light output from the light source 120. The adaptable lens 125 may allow for adjusting the divergence angle of the output laser light. The adaptable lens 125 may be adjusted so that, in the first configuration, the light source 120 generates collimated laser light. The collimated laser light may illuminate the region of the subject 10 to form the speckle pattern for the speckle vibrometry SV data. Further, the adaptable lens 125 may be adjusted so that, in the second configuration, the light source 120 generates divergent laser light. The divergent laser light may illuminate the region of the subject 10 to form the speckle pattern for the speckle plethysmography SPG data. In the present example, the adaptable lens 125 may be controlled to alternatingly be in the first or second configuration, so that the light source 120 alternatingly generates collimated or divergent laser light, for the SV data and SPG data, respectively. Part of the light forming the speckle patterns may be scattered and/or reflected back towards the system 100.

The system 100 further comprises an imaging detector 130. The imaging 130 in the present example is a single imaging detector. The imaging detector 130 is configured to acquire images of the speckle pattern representing speckle vibrometry SV data, and to acquire images of the speckle pattern representing speckle plethysmography SPG data.

To collect the light scattered and/or reflected back from the subject 10 to the system 100, the imaging detector 130 may comprise an imaging lens 135. The imaging lens 135 may be an adaptable imaging lens 135, allowing the focal length of the imaging lens 135 to be adjusted. The adaptable imaging lens 135 may be adjusted so that, in the first configuration, the imaging lens 135 is defocused with respect to the speckle pattern. Thus, in the first configuration, the imaging detector 130 may acquire images of the speckle pattern for the speckle vibrometry SV data. Further, the adaptable imaging lens 135 may be adjusted so that, in the second configuration, the imaging lens 135 is in focus with respect to the speckle pattern. Thus, in the second configuration, the imaging detector 130 may acquire images of the speckle pattern for the speckle plethysmography SPG data. The images of the SV data and the images of the SPG data may hence be acquired from substantially the same region of the subject 10. In the present example, the system 100 may be configured to alternatingly switch between the first and the second configurations, to alternatingly acquire images of the SV data and the SPG data, respectively.

The system 100 further comprises a processing unit 140. The images acquired by the imaging detector 130, i.e. the images of SV data and the images of SPG data, may be transferred to the processing unit 140. The processing unit 140 is configured to process the images of the speckle pattern from the imaging detector 130.

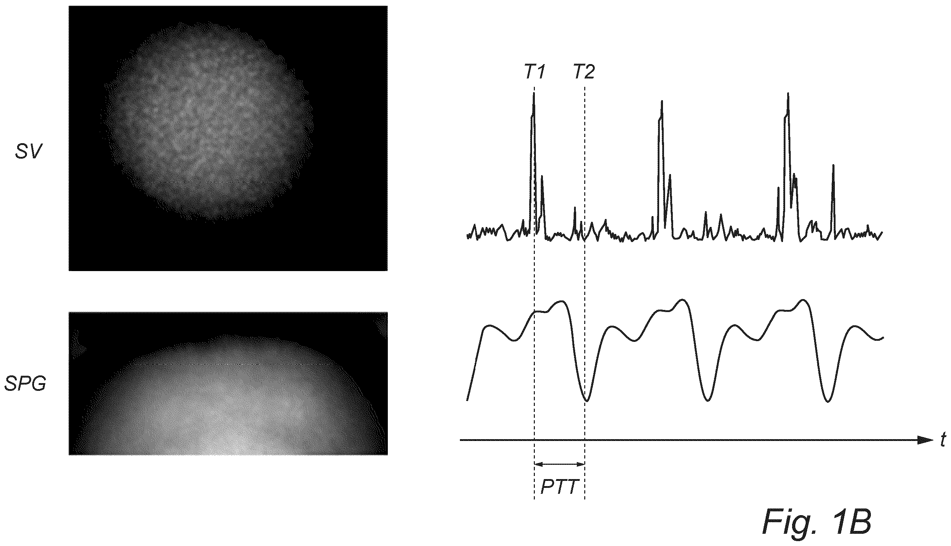

Fig. 1B schematically illustrates the data processing performed by the processing unit 140. The SV data is illustrated on the top row of Fig. 1B and the SPG data is illustrated on the bottom row of Fig. 1B.

The processing unit 140 is configured to extract the SV data from the images acquired in the first configuration of the system 100. An example of an SV image is illustrated at the top left of Fig. 1B. The image shows a defocused speckle pattern. The images of the defocused speckle pattern may be analyzed by the processing unit 140 to determine the speckles translational movement caused by vibrations, as a function of time. A graph illustrating the movements as a function of time is shown at the top right of Fig. 1B. When a flow of blood enters the aorta of the subject 10, the movement temporarily increases which is seen as sharp peaks in the graph. From this graph, the processing unit 140 may determine when a flow of blood enters the aorta of the subject 10. The time at which this occurs is referred to as the first time point, T1.

The processing unit 140 is configured to extract the SPG data from the images acquired in the second configuration of the system 100. An example of an SPG image is illustrated at the bottom left of Fig. 1B. The image shows a point in time when the speckle pattern is temporarily perturbed by the movement of the skin caused by the blood pulse arrival in the location of the artery system at the illuminated region. When this occurs, the speckle pattern may be blurred, which may result in reduced speckle contrast of the speckle pattern. A graph illustrating the speckle contrast as a function of time is shown at the bottom right of Fig. 1B. When the blood pulse arrives, there is a temporary change in the speckle contrast which is seen as peaks in the graph. From this graph, the processing unit 140 may determine when a blood pulse arrives in a location of the artery system of the subject 10 at the illuminated region. The time at which this occurs is referred to as the second time point, T2.

The processing unit 140 may be further configured to determine the PTT based on the first time point and the second time point. More specifically, in the present example, the processing unit 140 may be configured to determine the PTT by determining the difference between the first time point and the second time point. By using a combination of SV data and SPG data, PTT may be determined by monitoring a single region of the subject, without the requirement of monitoring at a proximal and at a distal region. Consequently, the system may be made to be more compact, since illumination and imaging of two separate regions being spatially separated by a large distance is not required. However, monitoring a proximal and a distal region is still possible, and such an approach may optionally be selected, as will be described below.

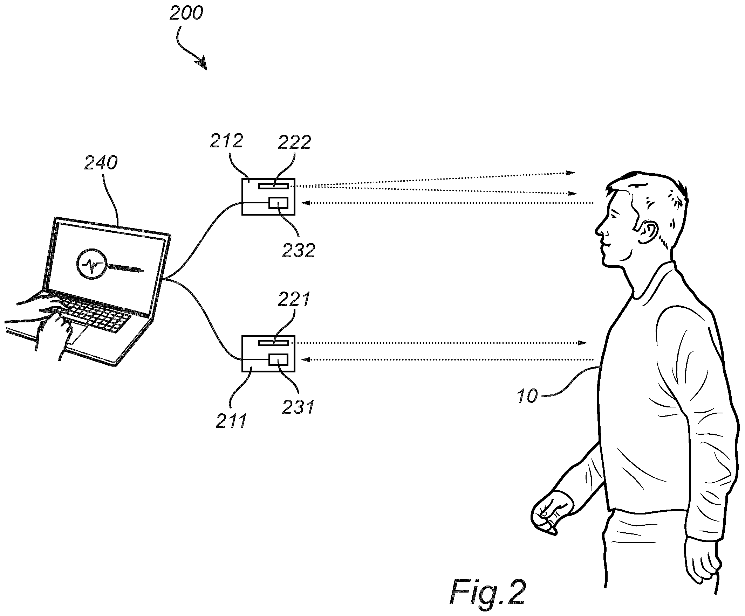

Fig. 2 schematically illustrates an alternative system 200 for determining a pulse transit time, PTT, of a subject 10. The system 200 may comprise a first subsystem 211 and a second subsystem 212.

The first subsystem 211 may comprise a first light source 221. The first light source 221 may be configured to generate collimated laser light for illuminating a first region of the subject 10. As the first region of the subject 10 is illuminated, the speckle pattern for the SV data may be formed. The first region may typically be a proximal region of the subject 10. Some of the light may be scattered and/or reflected back from the subject 10 to the first subsystem 211. The first subsystem 211 may further comprise a first imaging detector 231. The first imaging detector 231 may be configured to acquire images of the speckle pattern for the SV data. The first subsystem 211 may correspond to a first configuration of the system 200.

The second subsystem 212 may comprise a second light source 222. The second light source 222 may be configured to generate divergent laser light for illuminating a second region of the subject 10. As the second region of the subject 10 is illuminated, the speckle pattern for the SPG data may be formed. The second region may be spatially separated from the first region. The second region may typically be a distal region of the subject 10. Some of the light may be scattered and/or reflected back from the subject 10 to the second subsystem 212. The second subsystem 212 may further comprise a second imaging detector 232. The second imaging detector 232 may be configured to acquire images of the speckle pattern for the SPG data. The second subsystem 212 may correspond to a second configuration of the system 200.

The images for the SV data and the images for the SPG data may hence be acquired from two spatially separate regions of the subject 10. In the present example, the system 200 may be configured to simultaneously or alternatingly acquire images for the SV data and the SPG data.

It serves to mention that, although both the first subsystem 211 and the second subsystem 212 are here illustrated as being configured for sensing at a distance to the subject 10, it should be realized that the first subsystem 211 and/or the second subsystem 212 may alternatively be configured to be wearable on the body of the subject 10.

The system 200 further comprises a processing unit 240. In the present example, the processing unit 240 may be arrange in a computer being a separate unit from the first 211 and second 212 subsystems. The images acquired by the first 231 and second 232 imaging detectors, i.e. the images of SV data and the images of SPG data, may be transferred to the processing unit 240. The images may be transferred wirelessly, or as illustrated in Fig. 2 by a wired connection. The processing unit 240 may be configured to process the images of the speckle pattern from the first 231 and second 232 imaging detectors to determine the PTT based on the first time point and the second time point, in the same manner as described for the system 100 in relation to Fig. 1B.

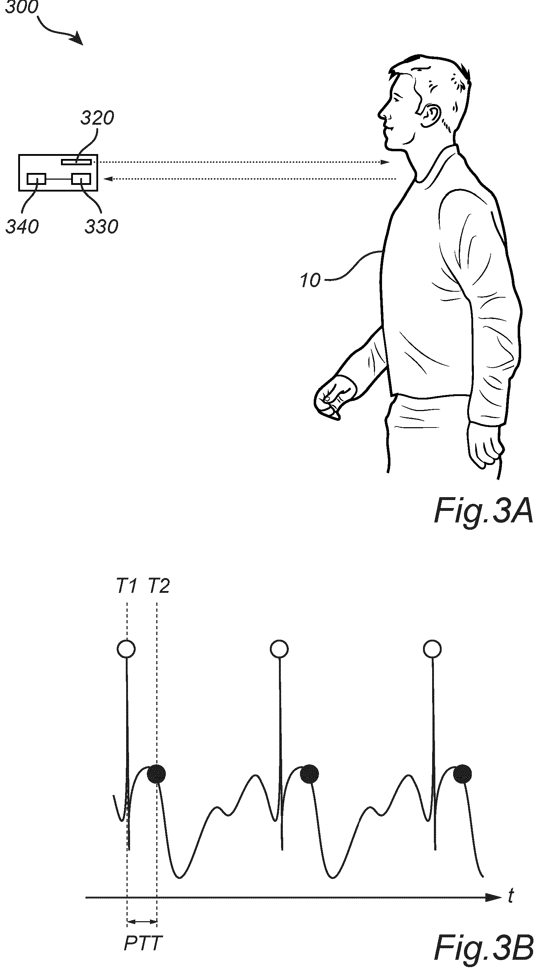

Fig. 3A schematically illustrates a system 300 for determining a pulse transit time, PTT, of a subject 10. The system 300 shares some of the features with the system 100 described in relation to Fig. 1A, the details of which are not repeated here.

The system 300 comprises a light source 320. In the present example, the light source 320 is configured to illuminate a single region of the subject 10, so as to form the speckle pattern for the SV data, and the speckle pattern for the SPG data in the same single region. Typically, this single region is a proximal region such as the chest region of the subject 10.

The system 300 comprises an imaging detector 330, being a single imaging detector. The light source 320 and the imaging detector 330 are configured to generate images representing both the SV data and the SPG data in the same image.

The system 300 further comprises a processing unit 340. The images acquired by the imaging detector 330, i.e. the images of both the SV data and the SPG data in the same image, may be transferred to the processing unit 340. The processing unit 340 is configured to process the images of the speckle pattern from the imaging detector 330.

Fig. 3B schematically illustrates data extracted, by the processing unit 340, from images comprising both the SV data and the SPG data in the same image.

When a flow of blood enters the aorta of the subject 10, the speckles translational movement temporarily increases which is seen as sharp peaks in the graph. From this graph, the processing unit 340 may determine when a flow of blood enters the aorta of the subject 10. The time at which this occurs is referred to as the first time point, T1.

When the blood pulse arrives, there is a temporary change in the speckle contrast which is seen as wider peaks in the graph. From this graph, the processing unit 340 may determine when a blood pulse arrives in a location of the artery system of the subject 10 at the illuminated region. The time at which this occurs is referred to as the second time point, T2.

It should be realized that the signal as illustrated in Fig. 3B, extracted from images comprising both SV data and SPG data, may appear similar to the signal extracted from images of SV data, as illustrated at the top right of Fig. 1B, superimposed onto the signal extracted from images of SPG data, as illustrated at the bottom right of Fig. 1B. The processing unit 340 may thus be configured to separate, or otherwise distinguish between the SV data and the SPG data. By way of example, said separation may be provided by filtering, such as low-pass, high-pass, and/or band-pass filtering, or the like.

By the present arrangement, the processing unit 340 may be able to distinguish between the SV part and the SPG part of the data, and thereby determine the first and second time points, T1 and T2. The processing unit 340 may be further configured to determine the PTT based on the first time point and the second time point, for example by determining the difference between the first time point and the second time point. By acquiring images comprising a combination of SV data and SPG data, PTT may be determined by monitoring a single region of the subject, and with a single set of images.

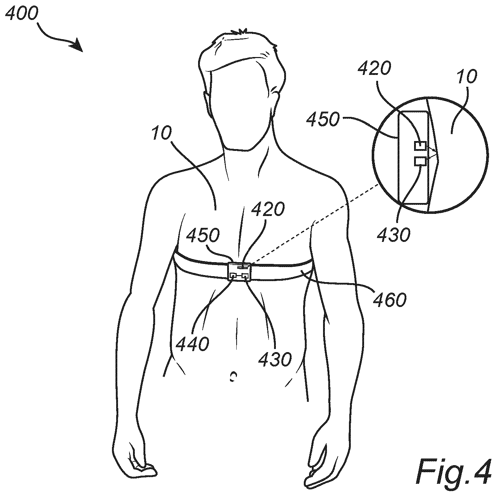

Fig. 4 schematically illustrates a wearable system 400 for determining a pulse transit time, PTT, of a subject 10. The system 400 comprises a housing 450. The housing may be attached to a strap 460. The strap 460 is configured to be arranged around the torso of the subject 10, such that the housing 450 is wearable on the body of the subject 10. By way of example, the housing 450 may be arranged on the chest of the subject 10. The strap may be made of for example a textile and/or a garment. By the present arrangement, the housing may be comfortably worn by the subject 10. By way of example, the system 400 may the system 100 as described in relation to Figs 1A-1B, or the system 300 described in relation to Figs 3A-3B.

The system 400 comprises a light source 420 configured to generate laser light for illuminating a region of the subject 10. The light source 420 is arranged in the housing 450 such that the laser light generated by the light source 420 is directed towards the skin on the chest of the subject 10, when the system 400 is attached to torso. By the present arrangement, the laser light may form a speckle pattern on the skin on the chest of the subject 10.

The system 400 further comprises an imaging detector 430, being a single imaging detector. The imaging detector 430 is arranged in the housing 460 in such a way that light being scattered and/or reflected by the skin on the chest of the subject 10 may impinge onto the imaging detector 430. By way of example, the imaging detector may comprise an imaging lens or or a microlens array (not shown here) in order to collect the light. In this manner, the light source 420 and the imaging detector 430 are configured to generate images of the speckle pattern representing the SV data and images the speckle pattern representing the SPG data.

The system 400 further comprises a processing unit 440. The images acquired by the imaging detector 430, i.e. the images of the SV data and the SPG data, may be transferred to the processing unit 440. The processing unit 440 is configured to process the images of the speckle pattern from the imaging detector 430. Given as non-limiting example, the processing unit 440 may be configure to process the images as described in relation to Fig. 1B and/or Fig. 3B.

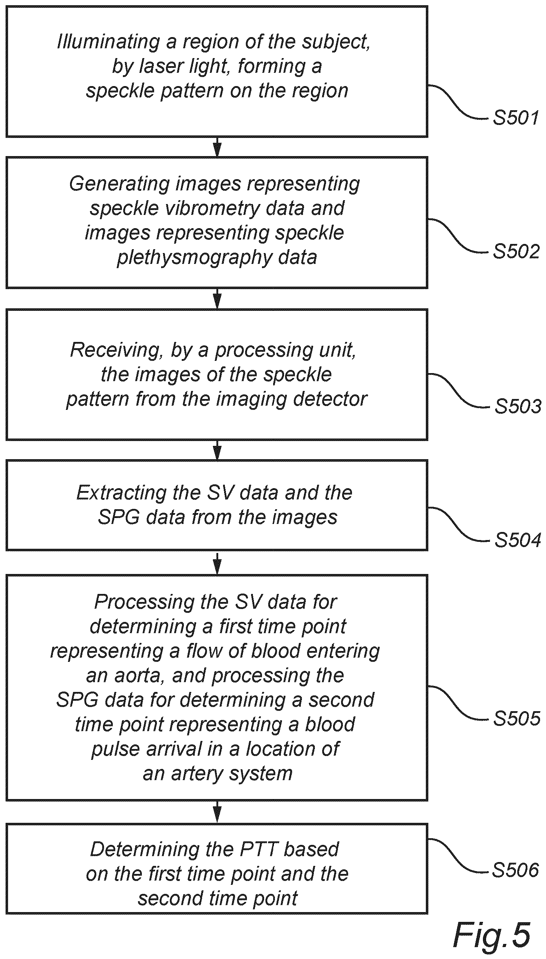

Fig. 5 illustrates a schematic block diagram shortly summarizing the method for determining a pulse transit time (PTT) of a subject. It should be understood that the steps of the method, although listed in a specific order herein, may be performed in any order suitable.

The method may comprise illuminating S501 at least one region of the subject, by laser light generated by at least one light source of a system. The laser light is configured to form a speckle pattern on the at least one region.

The method may comprise generating S502, by the at least one light source and at least one imaging detector, images representing speckle vibrometry (SV) data.

The method may comprise generating S502, by the at least one light source and the at least one imaging detector, images representing speckle plethysmography (SPG) data.

The method may comprise receiving S503, by a processing unit of the system, the images of the speckle pattern from the at least one imaging detector.

The method may comprise extracting S504, by the processing unit, the SV data from the images.

The method may comprise processing S505, by the processing unit, the SV data for determining a first time point representing a flow of blood entering an aorta.

The method may comprise extracting S504, by the processing unit, the SPG data from the images.

The method may comprise processing S505, by the processing unit, the SPG data for determining a second time point representing a blood pulse arrival in a location of an artery system associated with the at least one region.

The method may comprise determining S506, by the processing unit, the PTT based on the first time point and the second time point.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A system (100, 200, 300, 400) for determining a pulse transit time, PTT, of a subject (10), the system (100, 200, 300, 400) comprising:
at least one light source (120, 221, 222, 320, 420) configured to generate laser light for illuminating at least one region of the subject (10), wherein the laser light is configured to form a speckle pattern on the at least one region;
at least one imaging detector (130, 231, 232, 330, 430) configured to acquire images of the speckle pattern; and
a processing unit (140, 240, 340, 440) configured to receive and process the images of the speckle pattern from the at least one imaging detector (130, 231, 232, 330, 430);
wherein the at least one light source (120, 221, 222, 320, 420) and the at least one imaging detector (130, 231, 232, 330, 430) are configured to generate images representing speckle vibrometry, SV, data, and wherein the processing unit (140, 240, 340, 440) is configured to extract the SV data from the images and process the SV data for determining a first time point representing a flow of blood entering an aorta;
wherein the at least one light source (120, 221, 222, 320, 420) and the at least one imaging detector (130, 231, 232, 330, 430) are configured to generate images representing speckle plethysmography, SPG, data, and wherein the processing unit (140, 240, 340, 440) is configured to extract the SPG data from the images and process the SPG data for determining a second time point representing a blood pulse arrival in a location of an artery system associated with the at least one region; and
wherein the processing unit (140, 240, 340, 440) is further configured to determine the PTT based on the first time point and the second time point.

2. The system (100, 200, 400) according to claim 1, wherein the at least one light source (120, 221, 222, 420) and the at least one imaging detector (130, 231, 232, 430) are configured to generate the images representing the SV data, in a first configuration of the system (100, 200, 400), and
wherein the at least one light source (120, 221, 222, 420) and the at least one imaging detector (130, 231, 232, 430) are configured to generate the images representing the SPG data, in a second configuration of the system (100, 200, 400).

3. The system (100, 200, 400) according to claim 2, wherein, in the first configuration, the at least one light source (120, 221, 222, 420) is configured to generate collimated laser light for illuminating the at least one region of the subject to form the speckle pattern for the speckle vibrometry, SV data.

4. The system (100, 200, 400) according to any one of claims 2 or 3, wherein, in the second configuration, the at least one light source (120, 221, 222, 420) is configured to generate divergent laser light for illuminating the at least one region of the subject to form the speckle pattern for the speckle plethysmography, SPG data.

5. The system (100, 200, 400) according to any one of claims 2 to 4, wherein, in the first configuration, the at least one imaging detector (130, 231, 232, 430) comprises an imaging lens configured to be defocused with respect to the speckle pattern.

6. The system (100, 200, 400) according to any one of claims 2 to 5, wherein, in the second configuration, the at least one imaging detector (130, 231, 232, 430) comprises an imaging lens configured to be in focus with respect to the speckle pattern.

7. The system (100, 300, 400) according to any one of the preceding claims, wherein the at least one light source (120, 320, 420) is a single light source,
wherein the single light source (120, 320, 420) is configured to form the speckle pattern for the speckle vibrometry, SV, data, and
wherein the single light source (120, 320, 420) is configured to form the speckle pattern for the speckle plethysmography, SPG, data.

8. The system (100, 300, 400) according to any one of the preceding claims, wherein the at least one imaging detector (130, 330, 430) is a single imaging detector,
wherein the single imaging detector (130, 330, 430) is configured to acquire images representing speckle vibrometry, SV, data, and
wherein the single imaging detector (130, 330, 430) is configured to acquire images representing speckle plethysmography, SPG, data.

9. The system (200) according to any one of claims 2 to 8, wherein the at least one region comprises a first region and a second region, spatially separated from the first region, wherein, in the first configuration, the at least one light source (221, 222) and the at least one imaging detector (231, 232) are configured to generate images of the speckle pattern in the first region representing speckle vibrometry, SV, data, and wherein, in the second configuration, the at least one light source (221, 222) and the at least one imaging detector (231, 232) are configured to generate images of the speckle pattern in the second region representing speckle plethysmography, SPG, data.

10. The system (100, 300, 400) according to any one of claims 1 to 8, wherein the at least one region is a single region, wherein the at least one light source (120, 320, 420) and the at least one imaging detector (130, 330, 430) are configured to generate images of the speckle pattern in the single region representing speckle vibrometry, SV, data, and wherein the at least one light source (120, 320, 420) and the at least one imaging detector (130, 330, 430) are configured to generate images of the speckle pattern in the single region representing speckle plethysmography, SPG, data.

11. The system (100, 200, 300, 400) according to any one of the preceding claims, wherein the processing unit (140, 240, 340, 440) is configured to determine the PTT by determining a difference between the first time point and the second time point.

12. The system (100, 200, 300) according to any of the preceding claims, wherein the system (100, 200, 300) is configured for sensing at a distance and is configured to illuminate the at least one region of the subject (10) and to acquire images of the speckle pattern from a distance to the subject (10) in the interval of 0.1 m to 4 m.

13. The system (100, 200, 300, 400) according to any one of claims 1 to 11, further comprising a housing (450) configured to be wearable on a body of the subject (10), and wherein the at least one light source (120, 221, 222, 320, 420) and the at least one imaging detector (130, 231, 232, 330, 430) are arranged in or on the housing (450).

14. The system (300) according to claim 1, wherein the at least one light source (320) and the at least one imaging detector (330) are configured to generate images representing both the SV data and the SPG data in the same image, and wherein the processing unit (340) is configured to extract the SV data and the SPG data from the same image.

15. A method for determining a pulse transit time (PTT) of a subject, the method comprising:
Illuminating (S501) at least one region of the subject, by laser light generated by at least one light source of a system, wherein the laser light is configured to form a speckle pattern on the at least one region;
generating (S502), by the at least one light source and at least one imaging detector, images representing speckle vibrometry (SV) data;
generating (S502), by the at least one light source and the at least one imaging detector, images representing speckle plethysmography (SPG) data;
receiving (S503), by a processing unit of the system, the images of the speckle pattern from the at least one imaging detector;
extracting (S504), by the processing unit, the SV data from the images;
processing (S505), by the processing unit, the SV data for determining a first time point representing a flow of blood entering an aorta;
extracting (S504), by the processing unit, the SPG data from the images;
processing (S505), by the processing unit, the SPG data for determining a second time point representing a blood pulse arrival in a location of an artery system associated with the at least one region; and
determining (S506), by the processing unit, the PTT based on the first time point and the second time point.
